(19) **Europäisches Patentamt**
**European Patent Office**
**Office européen des brevets**

(11) **EP 3 252 019 A1**

(12) **EUROPEAN PATENT APPLICATION**
published in accordance with Art. 153(4) EPC

(43) Date of publication:
06.12.2017 Bulletin 2017/49

(21) Application number: 16779829.7

(22) Date of filing: 02.03.2016

(51) Int Cl.:
*C02F 3/00* (2006.01)     *C02F 3/10* (2006.01)
*C02F 3/12* (2006.01)     *C02F 3/34* (2006.01)

(86) International application number:
**PCT/JP2016/056340**

(87) International publication number:
**WO 2016/167037 (20.10.2016 Gazette 2016/42)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **13.04.2015 JP 2015081674**

(71) Applicant: **Fuji Electric Co., Ltd.
Kawasaki-shi, Kanagawa 210-9530 (JP)**

(72) Inventor: **TAGUCHI, Kazuyuki
Kawasaki-shi
Kanagawa 210-9530 (JP)**

(74) Representative: **MERH-IP Matias Erny Reichl
Hoffmann
Patentanwälte PartG mbB
Paul-Heyse-Strasse 29
80336 München (DE)**

(54) **METHOD FOR TREATING WASTEWATER, AND ACTIVATOR FOR TREATING WASTEWATER**

(57)     Provided are a method for treating wastewater and an activator for treating wastewater with which it is possible to maximize the utilization of microorganisms such as *Bacillus* bacteria in an activated sludge treatment of wastewater while minimizing treatment cost.

A method for treating wastewater in which wastewater including organic matter is introduced into a treatment tank and the wastewater is subjected to an activated sludge treatment by microorganisms in the treatment tank, wherein an activator containing a component for activating the microorganisms is added to the wastewater to be subjected to the activated sludge treatment in the treatment tank; and at least 50% (by quantity) of the entirety of the activator has a particle size of less than 10 $\mu$m. Also provided is an activator therefor. The microorganisms preferably include *Bacillus* bacteria.

EP 3 252 019 A1

**Description**

TECHNICAL FIELD

The present invention relates to a method for treating wastewater and to an activator for treating wastewater, the method and activator utilized to biodegrade wastewater containing organic matter, such as household wastewater or industrial wastewater, to water quality that meets wastewater standards.

BACKGROUND ART

Biological treatment such as the standard activated sludge process or oxidation ditch process is an extremely advantageous treatment method in terms of cost in wastewater treatment because the required equipment need not be as complex as that used in methods involving chemical or physical purification, few byproducts are generated, and less energy input is required. The standard activated sludge process in particular is widely used in economically developing emerging countries because large quantities of water can be treated in a short time and control is relatively easy.

Broadly speaking, a standard activated sludge process system comprises an aeration tank (treatment tank where biological treatment is carried out) and a settling tank. Aerobic conditions are established in the aeration tank by blowing air into the wastewater using a blower. Microorganisms that purify the wastewater of organic matter are activated, and the organic matter in the wastewater is removed. The activated sludge and treated water are separated in the settling tank. The treated water of the upper layer is then removed to the outside, and released into the environment after appropriate post-treatment. The activated sludge is sent back to the aeration tank to maintain the necessary microbial concentration in the aeration tank, and any excess (excess sludge) is removed to the outside and finally disposed of as industrial waste. The microorganisms in the aeration tank are the major component of the excess sludge, which is an aggregation of microorganisms that have grown using the organic matter in the wastewater as a substrate. Therefore, the volume of sludge generated also increases as the organic matter in the wastewater is removed

The microorganisms utilized to purify the wastewater in such a wastewater treatment process naturally predominate or are activated in the wastewater treatment environment. The food chain from various types of bacteria to protozoa also stretches, and the organic matter is removed. Removal of the organic matter in the wastewater therefore takes a relatively long time in biological treatment.

Improving wastewater treatment efficiency using a method of making *Bacillus* bacteria predominate has therefore been proposed and put to practical use, the *Bacillus* bacteria having a rapid proliferation rate even among the microorganisms in the wastewater treatment environment and producing large amounts of enzymes to decompose organic matter. Specifically, *Bacillus* bacteria are known to extracellularly secrete proteases, which are enzymes that degrade proteins; amylases, which are enzymes that degrade carbohydrates such as starch; lipases, which are enzymes that degrade fats; etc., in larger quantities than other bacteria. Sludge is composed of inorganic matter and macromolecular organic matter such as carbohydrates, proteins, and lipids. Given that *Bacillus* bacteria degrade and lower the molecular weight of these substances through their extracellular enzymes, incorporation thereof into *Bacillus* bacteria and other microorganisms within the treatment tank is promoted, the wastewater treatment speed is improved, and the amount of sludge generated is also decreased.

*Bacillus* bacteria are commonly known to predominate in the microbial flora balance of the activated sludge when minerals, etc., are added in the wastewater treatment process, which is presumably because silicon compounds included in the minerals promote the growth of *Bacillus* bacteria or improve tolerance in unfavorable environments (see Non-patent Document 1) .

As regards wastewater treatment utilizing *Bacillus* bacteria, the use of silicic acid, magnesium silicate, magnesium sulfate, etc. as an activator to induce and maintain the predominance of *Bacillus* bacteria in the treatment tank is described in, e.g., Patent Document 1 below.

However, the cost of such activators must be kept down to reduce the treatment cost, and it is desirable to use inexpensive materials such as materials derived from minerals rather than expensive reagents and formulations thereof. Patent Document 2 below describes, e.g., the use of silicic acid ions or magnesium ions eluted from minerals or processed products having a mineral as a raw material. Patent Document 3 below describes the use of silicon-containing substances such as cement, cement intermediate products, blast furnace slag, and coal ash as activators of *Bacillus* bacteria. The patent describes the use of these substances in the form of a silicon sol obtained by heat treatment in the presence of an alkaline substance and dissolution in an acid solvent.

[Prior Art Documents]

[Non-patent Documents]

[Non-patent Document 1] Journal of Environmental Biotechnology (2011), vol. 11, no. 1-2, pp. 47-53.

[Patent Documents]

[Patent Document 1] Japanese Laid-Open Patent Application No. 2002-113486
[Patent Document 2] Japanese Laid-Open Patent Application No. 2005-329301
[Patent Document 3] Japanese Laid-Open Patent Application No. 2012-5924

DISCLOSURE OF THE INVENTION

[Problems to Be Solved by the Invention]

However, mineral-derived materials, etc., are generally sparingly soluble materials. Even when they are added to the treatment tank as activators, the amount of the components thereof that can be utilized by microorganisms is very small, and it is difficult to say that a suitable amount appropriate for microorganisms such as *Bacillus* bacteria is being supplied. In addition, since addition of an excess of activator is related to an increase in the amount of sludge generated, a problem has been presented in that the sludge disposal cost rises, resulting in an increase in wastewater treatment cost.
In addition, silicon, which promotes the proliferation of *Bacillus* bacteria, etc., can be supplied in an ionic state by using the silicon sol described in Patent Document 3 (Patent Document 3, paragraph 0032, etc.), but a problem is presented in that costs mount merely through preparing the silicon sol. A risk is also presented in that, when used to prepare the silicon sol, residual alkalis and acids in the sol will negatively affect the microbial flora balance within the treatment tank.
It is an objective of the invention to solve the above problems and provide a method for treating wastewater and an activator for treating wastewater that make it possible to maximize the utilization of microorganisms such as *Bacillus* bacteria in the activated sludge treatment of wastewater while minimizing the treatment cost.

[Means to Solve the Problems]

To achieve the aforesaid objective, the method for treating wastewater of the present invention is a method for treating wastewater in which wastewater including organic matter is introduced into a treatment tank and the wastewater is subjected to an activated sludge treatment by microorganisms in the treatment tank, wherein the method for treating wastewater is characterized in that an activator containing a component for activating the microorganisms is added to the wastewater to be subjected to the activated sludge treatment in the treatment tank, and at least 50% (by quantity) of the entirety of the activator has a particle size of less than 10 $\mu$m.
In the method for treating wastewater of the present invention, the microorganisms preferably include *Bacillus* bacteria.
In addition, the activator preferably includes a silicon compound.
In addition, the activator preferably also includes at least one selected from the group consisting of an iron compound, a magnesium compound, a calcium compound, and a manganese compound.
In addition, the activator preferably is obtained by pulverizing at least one selected from the group consisting of blast furnace slag, diatomaceous earth, perlite, and cement.
In addition, the activator preferably is used by having a solubilizer of a silicon compound additionally combined with the activator.
In addition, the solubilizer preferably includes a cationic polymer for suppressing polymerization of silicon and/or a silicon compound.
The activator for treating wastewater of the present invention is an activator to be used in a method for treating wastewater in which wastewater including organic matter is introduced into a treatment tank and the wastewater is subjected to an activated sludge treatment by microorganisms in the treatment tank, wherein the activator for treating wastewater is characterized in containing a component for activating the microorganisms, at least 50% (by quantity) of the entirety of the activator having a particle size of less than 10 $\mu$m.
In the activator for treating wastewater of the present invention, the microorganisms preferably include *Bacillus* bacteria.

In addition, a silicon compound is preferably included as a component for activating the microorganisms.

In addition, at least one selected from the group consisting of an iron compound, a magnesium compound, a calcium compound, and a manganese compound is preferably also included as the component to activate the microorganisms.

In addition, the activator for treating wastewater preferably is obtained by pulverizing at least one selected from the group consisting of blast furnace slag, diatomaceous earth, perlite, and cement.

In addition, the activator for treating wastewater preferably is used in combination with a solubilizer of a silicon compound.

In addition, the solubilizer preferably includes a cationic polymer for suppressing polymerization of silicon and/or a silicon compound.

[Advantageous Effects of the Invention]

According to the present invention, since an activator having a predetermined particle size is added to wastewater that is to be subjected to activated sludge treatment in a treatment tank, components of the activator act effectively on the microorganisms such as *Bacillus* bacteria and promote their activation. Therefore, microorganisms such as *Bacillus* bacteria can be made to predominate without needlessly introducing an activator, and activated sludge treatment can be carried out more efficiently. The utilization of microorganisms such as *Bacillus* bacteria can thereby be maximized in the activated sludge treatment of wastewater while minimizing the treatment cost.

BRIEF DESCRIPTION OF THE DRAWINGS

FIG. 1 is a flow chart of wastewater treatment according to the present invention;

FIG. 2 is a procedure for measuring activity of a proteolytic enzyme (protease) included in wastewater in a treatment tank as an example of means for measuring enzyme activity derived from a microorganism such as *Bacillus* bacteria;

FIG. 3 is a procedure for measuring activity of a carbohydrate-degrading enzyme (amylase) included in the wastewater in the treatment tank as another example of means for measuring enzyme activity derived from a microorganism such as *Bacillus* bacteria;

FIG. 4 is a chart illustrating results of measuring the particle size distribution of pulverized blast furnace slag A in Test Example 1;

FIG. 5 is a chart illustrating results of measuring the particle size distribution of pulverized blast furnace slag B in Test Example 1;

FIG. 6 is a chart illustrating results of measuring the particle size distribution of a conventional, commercially available activator in Test Example 1;

FIG. 7 is a chart illustrating results of studying the effects of an activator on proteolytic enzyme production by *Bacillus* A in Test Example 3;

FIG. 8 is a chart illustrating results of studying the effects of an activator on proteolytic enzyme production by *Bacillus* B in Test Example 3;

FIG. 9 is a chart illustrating results of studying the effects of an activator on carbohydrate-degrading enzyme production by *Bacillus* A in Test Example 4;

FIG. 10 is a chart illustrating results of studying the effects of an activator on carbohydrate-degrading enzyme production by *Bacillus* B in Test Example 4;

FIG. 11 is a chart illustrating results of studying the effects of mineral components on proliferation of *Bacillus* A in Test Example 5;

FIG. 12 is a chart illustrating results of studying the effects of mineral components on proliferation of *Bacillus* B in Test Example 5;

FIG. 13 is a chart illustrating results of studying the effects of mineral components on proteolytic enzyme production by *Bacillus* A in Test Example 6;

FIG. 14 is a chart illustrating results of studying the effects of mineral components on proteolytic enzyme production by *Bacillus* B in Test Example 6;

FIG. 15 is a chart illustrating results of studying the effects of mineral components on carbohydrate-degrading enzyme production by *Bacillus* A in Test Example 7; and

FIG. 16 is a chart illustrating results of studying the effect of mineral components on carbohydrate-degrading enzyme production by *Bacillus* B in Test Example 7.

BEST MODE FOR CARRYING OUT THE INVENTION

There are no particular limitations as to the wastewater to be treated by the present invention as long as the wastewater contains organic matter. Examples include household wastewater and wastewater from cereal starch production industries, dairy products production industries, meat centers, sugar production industries, livestock food products production industries, livestock farming, meat products production industries, meat ham and sausage production industries, marine paste product production industries, marine food products production industries, organic chemical industrial production industries, inorganic chemical industrial production industries, etc.

In the wastewater treatment according to the present invention, there are no particular limitations as to the microorganisms that enable the activated sludge treatment as long as the microorganisms have the ability to purify the wastewater of organic matter included in the wastewater. Examples include *Bacillus* bacteria. *Bacillus* bacteria extracellularly secrete proteases, which are enzymes that degrade proteins; amylases, which are enzymes that degrade carbohydrates such as starches; lipases, which are enzymes that degrade fats; etc., and have a powerful ability to purify the wastewater of organic matter in wastewater. Therefore, the water quality of the treated water can be improved and wastewater can be treated more efficiently by making these microorganisms predominate within the treatment tank while minimizing the amount of sludge generated in association with wastewater treatment.

As described herein, "to predominate" means that the microorganisms are present in predominating amounts in the biological flora living within the treatment tank. Whether or not they predominate can be ascertained by randomly identifying the biological flora living within the treatment tank by, *inter alia,* determination of the 16Sr DNA sequence, and determining the proportion at which microorganisms belonging to the target genus are present relative to the other species. Specifically, the target-genus-microorganism count is preferably approximately $1 \times 10^7$ to $1 \times 10^{10}$ in 1 mL of activated sludge within the treatment tank.

These microorganisms may be present in the wastewater treatment process environment either naturally or having been made to predominate, but it is preferable to use microorganisms that can be supplied from outside the system and can live in the wastewater within the treatment tank. Specifically, *Bacillus methylotrophicus* CBMB205T (EU 194897), *Bacillus subtilis subsp. subtilis* DSM 10T (AJ 276351), *Bacillus subtilis subsp. subtilis* NBRC 3009, *Bacillus subtilis subsp. subtilis* ATCC 6051, etc., which are *Bacillus* bacteria, can be used.

Examples of a mode for supplying these microorganisms from outside the wastewater treatment process system include for supplying, using the *Bacillus* bacteria given as examples above as seed bacteria: addition to an introduced seed sludge, addition to the wastewater before the wastewater enters the treatment tank, and addition to the wastewater after the wastewater has entered the treatment tank. Adding these microorganisms at an early stage in the wastewater treatment operation makes it possible to ensure that they are in a predominant amount among the biological flora living within the treatment tank. Furthermore, in excess sludge obtained after wastewater treatment where the *Bacillus* bacteria given as examples above are made to predominate, the balance of biological flora in which these microorganisms predominate is maintained, and therefore such excess sludge may be obtained from another wastewater treatment facility and added as seed sludge when starting up a wastewater treatment facility anew to conduct treatment. Alternatively, in cases where the predominance of the *Bacillus* bacteria given as examples above is less prominent, such seed bacteria and seed sludge may be added whenever appropriate. A single species of microorganism may be supplied, or two or more species may be supplied.

An activator used in wastewater treatment according to the present invention is described below.

The activator may be any that contains a component that activates the above microorganisms that enable activated sludge treatment, but an activator that includes a silicon compound is especially preferred. Accordingly, the silicon component makes it possible to promote the growth of microorganisms such as *Bacillus* bacteria, improve tolerance in unfavorable environments, or bring about predominance within the treatment tank. Therefore, the water quality of the treated water can be improved and wastewater can be treated more efficiently while minimizing the amount of sludge generated in association with wastewater treatment.

The activator preferably also includes at least one selected from the group consisting of an iron compound, a magnesium compound, a calcium compound, and a manganese compound. Accordingly, as shown in the test examples described below, the iron component, magnesium component, calcium component, and/or manganese component promote(s) the proliferation of *Bacillus* bacteria and the secretion of proteolytic enzymes and carbohydrate-degrading enzymes from the *Bacillus* bacteria, and make(s) it possible to treat wastewater more efficiently.

The activator may be composed of, *inter alia,* a reagent containing the above components or a formulation thereof, but it is preferable to use, *inter alia,* industrial byproducts or materials derived from minerals, to lower the treatment cost. Examples include blast furnace slag, diatomaceous earth, perlite, and cement.

Blast furnace slag is a byproduct in a high-temperature furnace step for steel production, and includes components included in iron ore and components of the auxiliary raw materials limestone and coke. Blast furnace slag has been utilized in the past in cement, concrete, asphalt, ground improvement, fertilizers, etc. Blast furnace slag, as shown in the test examples described below, contains at least a silicon component, iron component, calcium component, magnesium component, aluminum component, and manganese component.

Diatomaceous earth is a fossil deposit of diatom shells, and has been utilized in the past in wall members, tile members, refractory bricks, filter members, deodorization, and other applications. Diatomaceous earth, as shown in the test examples described below, contains at least a silicon component, iron component, calcium component, magnesium component, aluminum component, and manganese component.

Perlite is calcined foam of volcanic rock that includes vitreous materials such as pearlite, obsidian, pitchstone, etc., and has been utilized in the past in soil improvement, insulation members, filter members, and fertilizers, etc. Perlite, as shown in the test examples described below, contains at least a silicon component, iron component, calcium component, magnesium component, aluminum component, and manganese component.

Cement has been used in the past as a material of concrete and mortar, and includes clinkers obtained by mixing and calcining limestone, clay, silica, iron, and other raw materials. Cement, as shown in the test examples described below, contains at least a silicon component, iron component, calcium component, magnesium component, and aluminum component.

Mineral-derived materials, etc., are commonly sparingly-soluble materials, and even when added to the treatment tank as activators, the amount of the components thereof that can be utilized by microorganisms is very small, and it is difficult to say that a suitable amount appropriate for microorganisms such as *Bacillus* bacteria is being supplied.

Therefore, an activator in which at least 50% (by quantity) of the entirety thereof has a particle size of less than 10 $\mu$m is used as the activator in the present invention. It is more desirable to use an activator in which at least 70% (by quantity) of the entirety thereof has a particle size of less than 10 $\mu$m, and even more desirable to use one in which at least 90% (by quantity) of the entirety thereof has a particle size less than 10 $\mu$m. By so controlling the particle size, the components of the activator act effectively on the microorganisms such as *Bacillus* bacteria and make it possible to promote the activation thereof. An activator having a predetermined particle size can be prepared, for example, by pulverization using an air flow-type grinder such as a jet mill or an impact-type grinder such as a power mill. An activator of the desired particle size may be sorted out by sieving as necessary after pulverization. Particle size distribution can be measured by a method such as estimating the particle size based on a scatter pattern created by light scattered when the particles are irradiated by laser light using a laser diffraction-scattering method. For example, the particle size distribution can be measured using a laser diffraction-scattering-type particle size distribution measuring instrument ("Microtrac MT300," Nikkiso Co., Ltd.).

An operational effect of an activator having the above predetermined particle size can be demonstrated in the present invention by thoroughly dispersing the individual particles within the treatment tank so that contact is made with the microorganisms in the activated sludge (mainly particles of a particle size of less than 10 $\mu$m: at least 50% (by quantity) of the entirety thereof having a particle size of less than 10 $\mu$m, preferably at least 70% (by quantity) of the entirety thereof having a particle size of less than 10 $\mu$m, more preferably at least 90% (by quantity) of the entirety thereof having a particle size of less than 10 $\mu$m. Therefore, the activator having the above predetermined particle size may, as appropriate, be dispersed in a liquid, granulated, or formed into tablets, or may be supplied in a non-pulverulent form, provided that there is no interference with such a mode of use.

The activator having the above predetermined particle size may be used in combination with other activated active substances. Accordingly, it is possible to compensate for substances lacking in components necessary for activation when using mineral-derived materials, etc. Examples of silicon compounds include silicon dioxide, sodium silicate, calcium silicate, and magnesium silicate. Examples of iron compounds include ferrous chloride, ferric chloride, ferrous sulfate, ferric sulfate, ferrous nitrate, and ferric nitrate. Examples of magnesium compounds include magnesium chloride, magnesium sulfate, magnesium hydroxide, and magnesium nitrate. Examples of calcium compounds include calcium chloride, calcium hydroxide, calcium oxide, and calcium nitrate. Examples of manganese compounds include manganese chloride, manganese sulfate, and manganese nitrate.

The activator having the above predetermined particle size may be used in combination with a solubilizer of a silicon compound. Accordingly, the silicon component of the activator acts even more effectively on microorganisms such as *Bacillus* bacteria and can promote activation thereof. The solubilizing effect of the solubilizer of the silicon compound is also remarkable with respect to an activator having the above predetermined particle size.

The solubilizer of the silicon compound is described below.

(1) Silicon compounds

Silicic acids and silicates are known forms of silicon compounds included in minerals, etc. Silicic acid is a generic term for compounds of silicon, oxygen, and hydrogen represented by the chemical formula $[SiO_x(OH)_{4-2x}]_n$. Forms confirmed in nature include orthosilicic acid ($H_4SiO_4$), metasilicic acid ($H_2SiO_3$), metadisilicic acid ($H_2Si_2O_5$), etc. Silicates are compounds having one or several silicon atoms as a core, and including an anion having a structure where an electronegative ligand incorporates the one or several silicon atoms therein. These compounds are also called silicates. Minerals in nature are present in large amounts as silicate minerals. The addition of a silicate mineral as a silicon supply source in a wastewater treatment process in which microorganisms such as *Bacillus* bacteria are made to predominate is straightforward and inexpensive. However, silicate minerals are insoluble because the silicon structure is present in polymerized form.

Specifically, olivine, garnets, etc. are called nesosilicate minerals and have an isolated tetrahedron of $[SiO_4]^{4-}$. Vesuvianite, epidotes, etc., are called sorosilicate minerals and have a double tetrahedral structure of $[Si_2O_7]^{6-}$. Beryl, tourmalines, etc., are called cyclosilicate minerals and have a ring structure of $[Si_nO_{3n}]^{2n-}$. Pyroxenes are called isosilicate minerals and have a single chain structure of $[Si_nO_{3n}]^{2n-}$. Amphiboles, etc., are called isosilicate minerals and have a structure of $[Si_{4n}O_{11n}]^{6n-}$, which is a double chain structure. Micas and clay minerals are called phyllosilicate minerals and have a sheet-like structure of $[Si_{2n}O_{5n}]^{2n-}$. Quartzes, feldspars, and zeolites are called tectosilicate minerals and have a three-dimensional network structure of $[Al_xSi_yO_{2(x+y)}]^{x-}$.

The silicon compounds included in minerals, etc. are polymers having the above structures. Therefore, lowering the molecular weight or monomerizing the polymer increases solubility in water and allows the silicon compounds to act effectively on microorganisms such as *Bacillus* bacteria.

Here, the polymerization of silicon advances, for example, by the continuous occurrence of the reaction shown in the following formula (1). Consequently, to suppress the polymerization of silicon and/or silicon compounds, it is effective to electrically neutralize the negative charge of $SiO^-$ that serves as the base point of the reaction.

$$SiO^- + Si(OH)_4 \rightarrow Si\text{-}O\text{-}Si(OH)_3 + OH^- \ ... \qquad (1)$$

(2) Solubilizer

There are no particular limitations as to the solubilizer as long as silicon compounds can be solubilized. Examples include cationic polymers. The positive charge of the cationic polymer electrically neutralizes the negative charge of $SiO^-$, making it possible to suppress the polymerization of silicon and/or silicon compounds, for example, via the above formula (1), and to thereby keep the silicon compound in a solubilized state in water or an aqueous solvent.

The cationic polymer is preferably a polymer having a cation such as a primary amine, secondary amine, tertiary amine, or quaternary ammonium. Concrete examples include allylamine hydrochloride polymers and allylamine hydrochloride-diallylamine hydrochloride copolymers, methyl diallylamine hydrochloride polymers, and diallyl dimethyl ammonium chloride polymers.

In order to cause the activator having the above predetermined particle size to act effectively on the microorganisms in the wastewater and activated sludge in the present invention, the activator having the above predetermined particle size is preferably contained in the following amounts (expressed in terms of the solids content) in a form in which the activator is contained: 50-100 mass%, more preferably 70-100 mass%, and even more preferably 90-100 mass%.

Embodiments of the present invention are further described below with reference to the drawings.

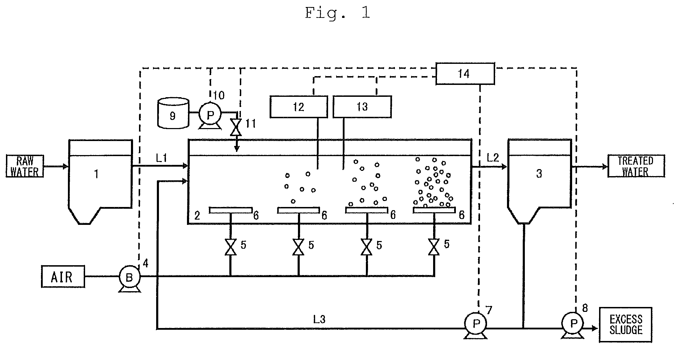

FIG. 1 illustrates a flow chart of wastewater treatment according to the present invention. First, wastewater including organic matter is introduced into a primary settling basin 1 as raw water. The raw water undergoes rough solid-liquid separation in this primary settling basin 1. Effluent from the primary settling basin 1 is sent to a treatment tank 2 through a pipe L1. The wastewater is biologically treated by *Bacillus* bacteria and other microorganisms in this treatment tank 2, and purified. At this time, the microorganisms proliferate as they assimilate organic matter, and form activated sludge. Effluent from the treatment tank 2 is sent to a settling tank 3 through a pipe L2. The activated sludge included in the effluent from the treatment tank 2 is precipitated in this settling tank 3. The supernatant in the settling tank 3 is released outside the system as treated water. Part of the sludge precipitated in the settling tank 3 is returned to the treatment tank 2 by a sludge return pump 7 through a pipe L3, and reused in wastewater treatment. The remaining sludge is sent as excess sludge to a sludge treatment facility, etc., by a sludge removal pump 8, and treated.

A plurality of diffuser plates 6 are provided in the lower part of the interior of the treatment tank 2 so that air can be supplied from a blower 4. Pipes leading to each diffuser plate are provided with an aeration adjustment valve 5 so that the aeration amount can be adjusted for each diffuser plate by opening and closing the valve. A measuring instrument 12 is also installed to measure the water quality of the wastewater within the treatment tank 2 so that the measurement values can be sent to a calculation unit 14.

In this embodiment, the amount of aeration from the diffuser plates 6 is adjusted so as to gradually increase from the side where the raw water is supplied (left side in FIG. 1; referred to as "wastewater supply side" hereinafter) toward the side where the treated water is discharged (right side in FIG. 1; referred to as "wastewater discharge side" hereinafter) so that an anaerobic region and an aerobic region are formed continuously within the treatment tank 2. The wastewater within the treatment tank 2 is made to flow slowly from the wastewater supply side toward the wastewater discharge side by a gradient in the amount of aeration from the diffuser plates 6 and/or by a stirring apparatus (not shown). Therefore, the wastewater introduced into the treatment tank 2 is exposed first to anaerobic conditions in the anaerobic region within the treatment tank 2, then, after a predetermined period of time, exposed to aerobic conditions for a predetermined period of time in the aerobic region within the treatment tank 2. Treatments for utilizing the activity of microorganisms that tend to prefer anaerobic conditions (denitrifying bacteria, dephosphorizing bacteria, denitrifying phosphorus-accumulating bacteria, etc.) and treatments for utilizing the activity of microorganisms that tend to prefer aerobic conditions (nitrifying bacteria, yeasts, *Escherichia coli,* etc.) can thereby be carried out efficiently within the treatment tank 2.

The anaerobic/aerobic conditions within the treatment tank 2 may also be established by another mode. For example, the entire treatment tank may be placed under anaerobic conditions for a predetermined period of time, whereupon the entire treatment tank is placed under aerobic conditions so that the wastewater within the treatment tank 2 is exposed to anaerobic/aerobic conditions respectively for a predetermined period of time, making it possible to conduct efficient biological treatment utilizing the activity of microorganisms that tend to prefer anaerobic conditions and the activity of microorganisms that tend to prefer aerobic conditions.

The water quality, such as the dissolved oxygen content, oxidation-reduction potential, hydrogen ion concentration, and sludge concentration serves as an indicator for monitoring the wastewater within the treatment tank. The measuring instrument 12 represents a measuring instrument used in monitoring by such conventional indicators.

The dissolved oxygen content is an indicator for assessing whether the required oxygen content is excessive or insufficient when the microorganisms in the activated sludge assimilate organic matter as a substrate. Therefore, if, on the basis of the dissolved oxygen content value measured by the measuring instrument 12, it is assessed in the calculation unit 14 that the dissolved oxygen in the treatment tank water is insufficient, a signal from the calculation unit 14 increases the operation level of the blower 4 or opens the aeration adjustment valve 5, or both, making it possible to exert control to increase the aeration volume of the treatment tank. If the dissolved oxygen is assessed to be excessive, a signal from the calculation unit 14 reduces the operation level of the blower 4 or closes the aeration adjustment valve 5, or both, making it possible to exert control to reduce the aeration amount of the treatment tank. Aeration may be stopped completely in the control of the latter case, as shall be apparent. Typically, it is preferable for the dissolved oxygen content under aerobic conditions to be managed so as to fall within a range of 2.0 to 3.5 mg/L.

The oxidation-reduction potential and the hydrogen ion concentration are indicators relating to removal of nitrogen from the wastewater within the treatment tank. Specifically, the nitrogen content of the wastewater is oxidized from ammonia ions into nitrite ions and nitrate ions by nitrifying bacteria, etc., and then converted into nitrogen by denitrifying bacteria, etc. As a result, the nitrogen content of the wastewater released from the treatment tank in a gas phase in the form of nitrogen gas, and thereby removed. The liquid becomes acidic when the ammonia ions are oxidized into the nitrite ions and nitrate ions. In addition, the denitrifying bacteria that reduce the nitrate ions into nitrogen gas are known to function anaerobically. Therefore, monitoring the anaerobic degree of the wastewater in the treatment tank according to the oxidation-reduction potential or hydrogen ion concentration serves as an indicator for assessing whether the nitrogen in the wastewater has been removed properly. Consequently, if, on the basis of the oxidation-reduction potential or hydrogen ion concentration values measured by the measuring instrument 12, it is assessed in the calculation unit 14 that the anaerobic degree of the treatment tank water is insufficient, a signal from the calculation unit 14 reduces the operation level of the blower 4 or closes the aeration adjustment valve 5, or both, making it possible to exert control to lower the aeration amount of the treatment tank. Aeration may be stopped completely in this case, as shall be apparent. If it is desired to cancel the anaerobic conditions, a signal from the calculation unit 14 increases the operation level of the blower 4 or opens the aeration adjustment valve 5, or both, making it possible to exert control to increase the aeration amount of the treatment tank. Typically, it is preferable to manage the oxidation-reduction potential to fall within a range of -150 to -200 mV under anaerobic conditions. The pH is preferably managed so as to be close to neutral; i.e., in a range of 6.5-7.

The sludge concentration is regarded to be the amount of microorganisms involved in the biological treatment of the wastewater. Since the organic matter in the wastewater assimilates more quickly in correspondence with an increase in microorganism content of the activated sludge, increasing the sludge concentration will also raise the treatment efficiency. However, since the amount of microorganisms increases accordingly when the sludge concentration is high, the aeration amount need to be raised, or the sludge need to be dissolved by auto-oxidation. In addition, organic matter must be introduced stably so that the sludge necessary for treatment is furnished by the total amount of sludge in the treatment tank. A balance between the sludge load and the organic matter load therefore becomes important to stably maintain the efficiency of the biological treatment of the wastewater. Consequently, if, on the basis of the sludge concentration value measured by the measuring instrument 12, it is assessed in the calculation unit 14 that the sludge load is excessive, a signal from the calculation unit 14 reduces the operation level of the sludge return pump 7 or increases the operation level of the sludge removal pump 8, or both, making it possible to exert control to lower the amount of sludge returned. Furthermore, sludge return may be stopped completely in this case, as shall be apparent. If the organic matter load is assessed to be excessive relative to the sludge load, a signal from the calculation unit 14 increases the operation level of the sludge return pump 7, or lowers the operation level of the sludge removal pump 8, or both, making it possible to exert control to increase the amount of sludge returned. Typically, the activated sludge concentration (MLSS) of the wastewater within the treatment tank 2 is managed to a range of 2000 to 2500 mg/L.

In the present invention, the activator described above is added to the wastewater within the reaction tank 2 from an activator supply tank 9 through an activator injection pump 10 and an activator injection adjustment valve 11 in such a wastewater treatment process. There are no particular limitation as to when the activator is to be added; a predetermined amount may be added continuously or intermittently at fixed intervals, or the desired mount may be added at any time. In addition, when the predominance of microorganisms such as *Bacillus* bacteria is less prominent; e.g., when a target number of microorganisms to be present in the wastewater within the reaction tank 2 has fallen below a predetermined threshold value, a predetermined amount of activator may be added. Alternatively, when the organic matter concentration of the wastewater to be treated within the treatment tank 2 has exceeded a predetermined threshold value, a predetermined amount of activator may be added.

In addition, in the embodiment shown in FIG. 1, enzyme activity derived from the microorganisms such as *Bacillus* bacteria included in the wastewater within the treatment tank 2 can be measured, and the biological treatment of the wastewater within the treatment tank 2 can be controlled based on the enzyme activity measured, in addition to or in place of the control described above. An enzyme activity measuring instrument 13 is therefore installed, the enzyme activity measuring instrument 13 being used to measure the enzyme activity derived from the microorganisms such as *Bacillus* bacteria included in the wastewater within the treatment tank 2, and the measurement value is sent to the calculation unit 14.

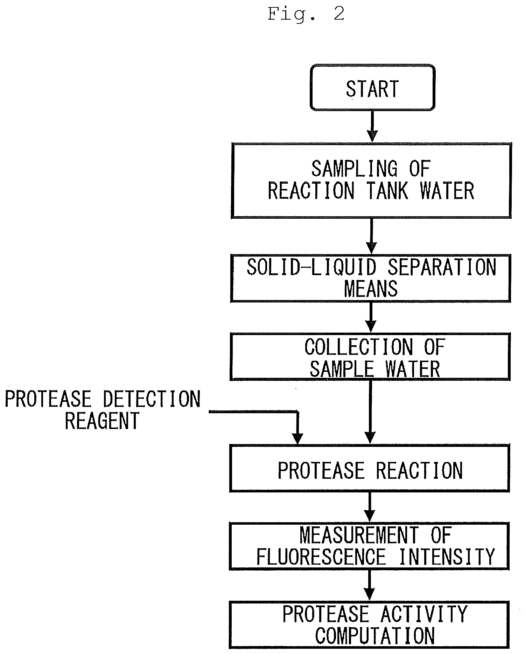

FIG. 2 illustrates a procedure for measuring the activity of a proteolytic enzyme (protease) included in the wastewater within the treatment tank 2 as an example of means for measuring the enzyme activity derived from the microorganisms such as *Bacillus* bacteria in the wastewater treatment according to the present invention. First, the treatment tank water is collected, and sample water is collected by solid-liquid separation means. Examples of the solid-liquid separation means include filtration by a filter having a pore size of 0.2 μm and centrifugation. Next, a protease detection reagent is added to the sample water, and a protease reaction is carried out for a predetermined period of time. For example, a fluorescent proteolytic enzyme assay kit (Thermo Fisher Scientific Inc.), etc., can be used as the protease detection reagent. This kit includes a reagent for which fluorescence intensity increases due to enzyme activity by proteolytic enzymes. When this kit is used, the fluorescence intensity is measured after standing for 5-60 minutes at room temperature after addition of the reagent, and protease activity in the sample water can be computed from a calibration curve, etc., produced in advance. Units such as micromoles per minute (unit), which is standardized by the amount of a predetermined degradation product produced per a period of time when a reference protein is degraded under predetermined conditions, can be used as an activity unit of protease activity.

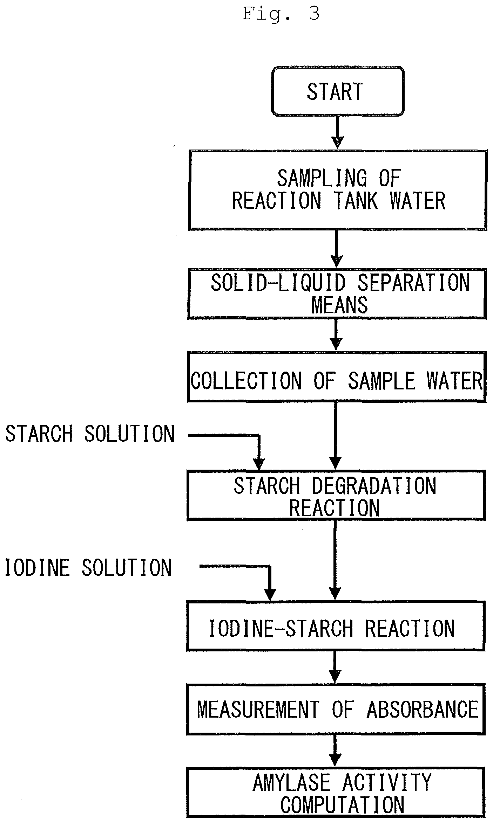

FIG. 3 illustrates an example of a procedure for measuring the activity of carbohydrate-degrading enzyme (amylase) included in the wastewater within the treatment tank 2 as another example of means for measuring the enzyme activity derived from microorganisms such as *Bacillus* bacteria in the wastewater treatment according to the present invention. First, the treatment tank water is collected, and the sample water is collected by solid-liquid separation means. Examples of the solid-liquid separation means include filtration by a filter having a pore size of 0.2 μm and centrifugation. Next, a water-soluble starch is added to the sample water, and a starch degradation reaction is carried out at room temperature for a predetermined period of time; typically about 60 minutes. Iodine solution is then added dropwise, and an iodine-starch reaction is conducted, whereupon the absorbance of the sample is measured. If starch remains, the sample turns a purple characteristic of an iodine-starch reaction; if the starch has been degraded, the sample does not change color. Coloration due to the iodine-starch reaction can be measured, for example, at an absorbance of 550 nm. The amylase activity can be computed from the measurement value. Units such as micromoles per minute (unit), which is standardized by the amount of a predetermined degradation product produced per a period of time when a standard water-soluble starch is degraded under predetermined conditions, can be used as the activity unit of amylase activity.

Furthermore, enzyme activity due to enzymes other than those mentioned above may serve as an indicator of microorganisms such as *Bacillus* bacteria. For example, enzyme activity due to lipolytic enzymes and cellulolytic enzymes, can also serve as an indicator.

The enzyme activity derived from microorganisms such as *Bacillus* bacteria included in the wastewater within the treatment tank is regarded to be a direct indicator reflecting the activity of the microorganisms such as *Bacillus* bacteria utilized in the biological treatment of the wastewater. Therefore, if, based on an enzyme activity value measured by the enzyme activity measuring instrument 13, the activity of the microorganisms such as *Bacillus* bacteria in the treatment tank water is assessed in the calculation unit 14 to be insufficient, a signal from the calculation unit 14 increases the operation level of the activator injection pump 10, or opens the activator injection adjustment valve 11, or both, making it possible to exert control to increase the amount of activator supplied to the wastewater within the treatment tank 2. If the activity of the microorganisms such as *Bacillus* bacteria is assessed to be sufficient, a signal from the calculation unit 14 decreases the operation level of the activator injection pump 10, or closes the activator injection adjustment valve 11, or both, making it possible to exert control to lower the amount of minerals supplied to the wastewater within the treatment tank 2. The supply of the activator may be stopped completely when the latter control is performed, as shall be apparent.

EXAMPLES

The present invention is described more concretely below through examples. However, the scope of the invention is in no way limited by these examples.

<Preparation Example 1> (Pulverized blast furnace slag A)

Blast furnace slag was pulverized by a jet mill (manufactured by Aisin Nano Technologies Co., Ltd.), and a powder of the blast furnace slag was prepared as the activator used in the activated sludge treatment. This is designated as pulverized blast furnace slag A below.

<Preparation Example 2> (Pulverized blast furnace slag B)

A powder of blast furnace slag was prepared by making the pulverization conditions weaker than in Preparation Example 1. The resulting powder is designated as pulverized blast furnace slag B below.

<Test Example 1> (Measurement of particle size distribution)

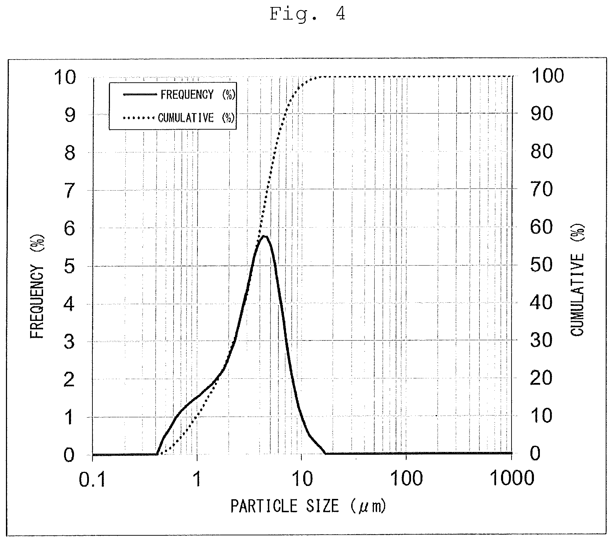

The particle size distribution of pulverized blast furnace slag A was measured using a laser diffraction-scattering-type particle size distribution measuring instrument ("Microtrac MT300," Nikkiso Co., Ltd.). The results are illustrated in FIG. 4. As illustrated in FIG. 4, at least 50% (by quantity) of the particles of the pulverized slag A had a particle size of less than 10 $\mu$m.

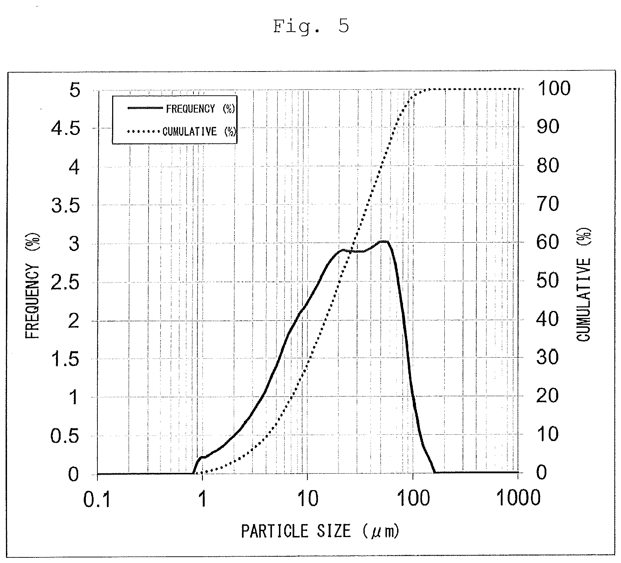

The particle size distribution of the pulverized blast furnace slag B was measured in the same manner. The results are illustrated in FIG. 5. As illustrated in FIG. 5, at least 50% (by quantity) of the particles of the pulverized slag B had a particle size exceeding 63 $\mu$m.

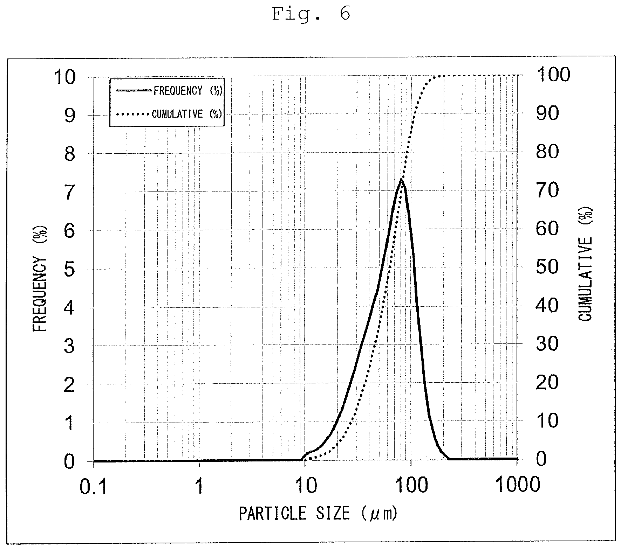

The particle size distribution of a commercial product marketed as an activator to be used in activated sludge treatment was also measured in the same manner. The results are illustrated in FIG. 6. As illustrated in FIG. 6, at least 50% (by quantity) of the particles in the number-based particle size distribution of the commercial product had a particle size that exceeded 23 $\mu$m.

<Test Example 2> (Effect of activator on proliferation of *Bacillus* bacteria)

A study was made of the effect, in activating the growth of *Bacillus* bacteria, of the pulverized blast furnace slag A, pulverized blast furnace slag B, and the commercial product marketed as an activator to be used in activated sludge treatment (major composition: 19.3 mass% of silicon, 3.6 mass% of iron, 3.9 mass% of calcium, 4.2 mass% of magnesium, 3.4 mass% of aluminum, and 0.1 mass% of manganese). *Bacillus* bacteria belonging to *Bacillus methylotrophicus* (referred to hereinafter as "*Bacillus* A") and belonging to *Bacillus subtilis* (referred to hereinafter as "*Bacillus* B") were used as *Bacillus* bacteria.

Nutrient media were respectively inoculated with each *Bacillus,* and shake culturing was carried out at 30°C. Culturing at this time was conducted by adding, as the activator for improving the activity of the *Bacillus* bacteria, the pulverized slag A, the pulverized slag B, or the commercial product to the media to make 16 mg/L each. Turbidity (OD 600) of a culture broth was measured over time after the start of culture. Doubling time of the *Bacillus* bacteria was computed from a proliferation curve.

Table 1 gives the results by *Bacillus* A.

[Table 1]

| (*Bacillus* A) | | | |
|---|---|---|---|
| | Pulverized slag A | Pulverized slag B | Commercial product |
| OD 600 after 8 hr culturing | 0.95 | 0.28 | 0.58 |
| Doubling time (hr) | 2.2 | n.d.* | 2.9 |
| Growth rate (1/hr) | 0.45 | n.d.* | 0.35 |
| n.d.*: not tested | | | |

Table 2 gives the results by *Bacillus* B.

[Table 2]

| (*Bacillus* B) | | | |
|---|---|---|---|
| | Pulverized slag A | Pulverized slag B | Commercial product |
| OD 600 after 8 hr culture | 1.15 | 0.25 | 0.55 |
| Doubling time (hr) | 2.1 | n.d.* | 3.3 |
| Growth rate (1/hr) | 0.47 | n.d.* | 0.33 |
| n.d.*: not tested | | | |

In the results, the effect on activating the proliferation of each *Bacillus* was high-to-low in the order of pulverized slag A, commercial product, pulverized slag B. A comparison of pulverized slag A and the commercial product according to the doubling time or growth rate revealed a difference of 1.3 times with *Bacillus* A and 1.6 times with *Bacillus* B, and the pulverized slag A had a higher activating effect on the proliferation of *Bacillus* bacteria than did the commercial product.

<Test Example 3> (Effect of activator on proteolytic enzyme production by *Bacillus* bacteria)

The effect of the pulverized blast furnace slag A, pulverized blast furnace slag B, and the commercial product marketed as an activator to be used in activated sludge treatment on activating proteolytic enzyme production by *Bacillus* bacteria was studied. *Bacillus* A and *Bacillus* B were used in the same way as in Test Example 2 as the *Bacillus* bacteria.
Nutrient media were respectively inoculated with each *Bacillus,* and shake culturing was carried out at 30°C. Culturing at this time was conducted by adding, as the activator for improving the activity of the *Bacillus* bacteria, the pulverized slag A, the pulverized slag B, or the commercial product to the media to make 16 mg/L each. Culturing was stopped 24 hours after starting, and the proteolytic activity was measured in a culture broth from which the *Bacillus* had been removed by centrifugation. It is noted that proliferation was found to reach saturation 24 hours after the start of culture.
The proteolytic activity was measured using a fluorescent proteolytic enzyme assay kit (Thermo Fisher Scientific Inc.). The reagent included in this kit is one for which the fluorescence intensity increases due to the presence of proteolytic enzymes. The fluorescence intensity of the samples was measured by supplying the samples to the kit. Separately trypsin, which is a proteolytic enzyme, was diluted stepwise at predetermined concentrations, and the fluorescence intensity was measured in the same manner to thereby produce a calibration curve. The proteolytic enzyme activity in the samples was determined as a trypsin equivalent per milliliter of culture broth by means of fitting the fluorescence intensity measured for each sample to the calibration curve.

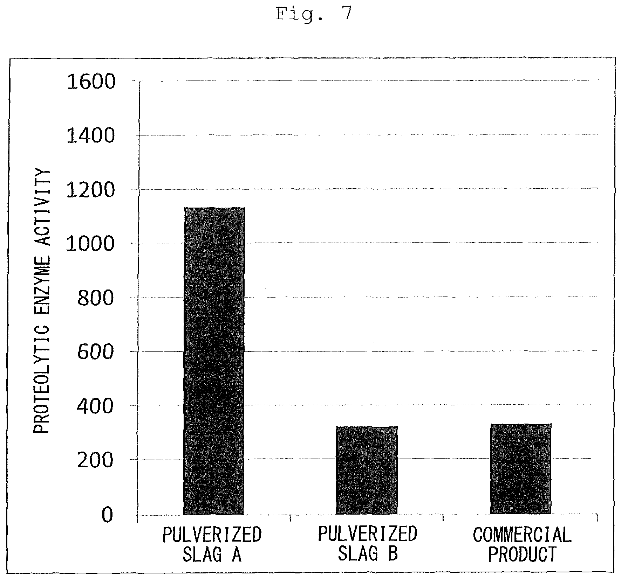

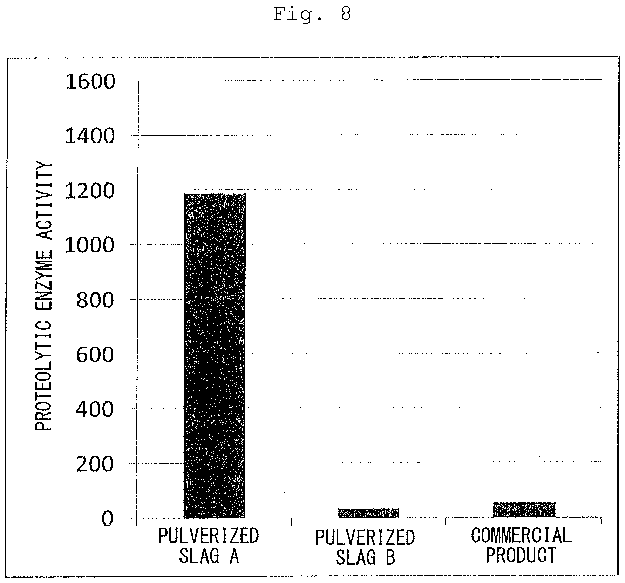

FIG. 7 illustrates the results obtained with *Bacillus* A. FIG. 8 illustrates the results obtained with *Bacillus* B.
As illustrated in FIG. 7, the trypsin equivalent of the proteolytic enzyme in the culture broth of *Bacillus* A was 1130 ng/mL with the pulverized slag A, 320 ng/mL with the pulverized slag B, and 330 ng/mL with the commercial product. Approximately three times more proteolytic enzyme was produced when the pulverized slag A was added to the medium than when the pulverized slag B and the commercial product were added to the medium in the same concentration.
Similarly, as illustrated in FIG. 8, the trypsin equivalent of the proteolytic enzyme in the culture broth of *Bacillus* B was 1190 ng/mL with the pulverized slag A, 33 ng/mL with the pulverized slag B, and 54 ng/mL with the commercial product. Approximately 22 times more proteolytic enzyme was produced when the pulverized slag A was added to the medium than when the commercial product was added to the medium in the same concentration. Approximately 36 times more proteolytic enzyme was produced than when the pulverized slag B was added to the medium in the same concentration.

<Test Example 4> (Effect of activator on carbohydrate-degrading enzyme production by *Bacillus* bacteria)

The activity of carbohydrate-degrading enzymes secreted in culture broth was studied in the respective culture broths of each *Bacillus* cultured in the same way as in Test Example 3.
Specifically, the culture broth was recovered 24 hours after the start of culturing, and culture broth from which the *Bacillus* had been removed by centrifugation was collected. A quantity of 0.05 mL of 0.5% water-soluble starch was added to 1.95 mL of this culture broth, and iodine solution was added dropwise after 60 minutes. If starch remains, the sample turns a bluish-purple that is characteristic of an iodine-starch reaction; if the starch has been degraded, the sample does not change color. The coloration was measured according to an absorbance of 550 nm (A 550).
The activity of carbohydrate-degrading enzymes was expressed in terms of the percentage of added starch that can be degraded in 60 minutes, as illustrated by the following calculation formula, and standardized.

$$\text{[Carbohydrate-degrading enzyme activity (\%)]} = \{([\text{A 550 of starch alone}] - [\text{A 550 of system with culture broth added}])/([\text{A 550 of starch alone}] - [\text{A 550 without starch}])\} \times 100$$

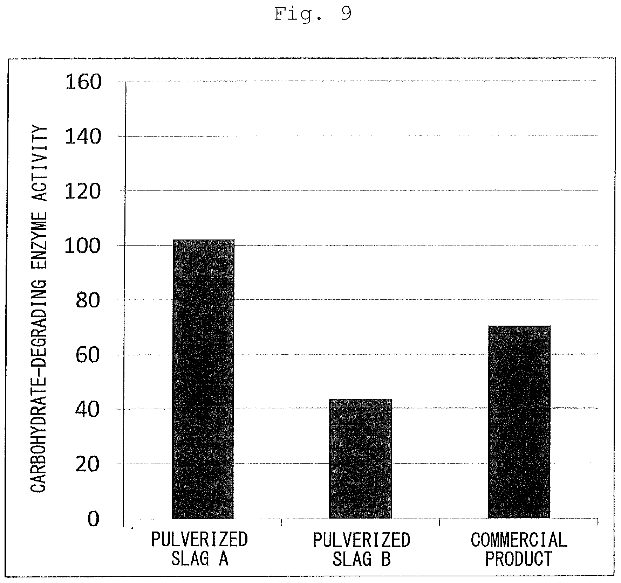

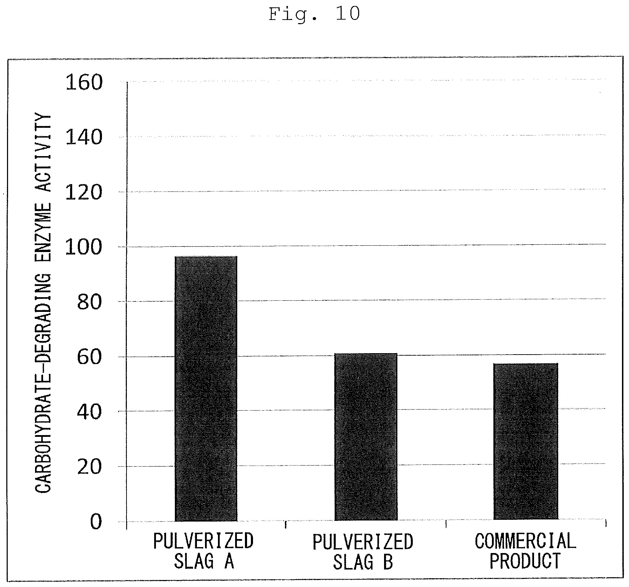

FIG. 9 illustrates the results obtained with *Bacillus* A. FIG. 10 illustrates the results obtained with *Bacillus* B.

As illustrated in FIG. 9, the carbohydrate-degrading enzyme activity in the culture broth of *Bacillus* A was 102% with the pulverized slag A, 44% with the pulverized slag B, and 70% with the commercial product. Approximately 1.5 times more carbohydrate-degrading enzymes were produced when the pulverized slag A was added to the medium than when the commercial product was added to the medium in the same concentration. Approximately 2.3 times more carbohydrate-degrading enzymes were produced than when the pulverized slag B was added to the medium in the same concentration.

Similarly, as illustrated in FIG. 10, the carbohydrate-degrading enzyme activity in the culture broth of *Bacillus* B was 96% with the pulverized slag A, 61% with the pulverized slag B, and 57% with the commercial product. Approximately 1.7 times more carbohydrate-degrading enzymes were produced when the pulverized slag A was added to the medium than when the commercial product was added to the medium in the same concentration. Approximately 1.6 times more carbohydrate-degrading enzymes were produced than when the pulverized slag B was added to the medium in the same concentration.

As validated by the above Test Examples 1-4, it is clear that, when a sparingly-soluble material such as blast furnace slag is used as an activator to be used in the activated sludge treatment, using the sparingly-soluble material pulverized to a predetermined particle size causes the activating effects involving *Bacillus* bacterial proliferation and enzyme activity to become more prominent. These effects were also superior to those of a conventional activator.

<Test Example 5> (Effect of mineral components on proliferation by *Bacillus* bacteria)

*Bacillus* bacteria are commonly known to predominate in the microbial flora balance of the activated sludge when minerals, etc., are added in a wastewater treatment process. This is presumably because the silicon compounds included in the minerals promote the growth of *Bacillus* bacteria and improve tolerance in unfavorable environments (Journal of Environmental Biotechnology (2011), vol. 11, no. 1-2, pp. 47-53, Abstracts of the 65th Meeting of the Society for Biotechnology, Japan, p. 221).

Therefore, iron, aluminum, calcium, magnesium, and manganese were selected as metal components other than silicon commonly included in minerals, and the effect of each on the proliferation of *Bacillus* bacteria was studied. *Bacillus* A and *Bacillus* B were used as *Bacillus* bacteria in the same way as in Test Examples 2-4.

Minimal media (5 g/L of glucose, 14 mg/L of $K_2HPO_4$, 6 g/L of $KH_2PO_4$, and 1 g/L of $NH_4Cl$) were respectively inoculated with each *Bacillus,* and shake culturing was carried out at 30°C. Silicon (final concentration: 27.8 mg/L), iron (final concentration: 9 mg/L), aluminum (final concentration: 8.8 mg/L), calcium (final concentration: 17.2 mg/L), magnesium (final concentration: 18 mg/L), and manganese (final concentration 20 mg/L) were added to the media as metal components to yield the final concentration shown in parentheses. A system to which no specific metal components were added was also established.

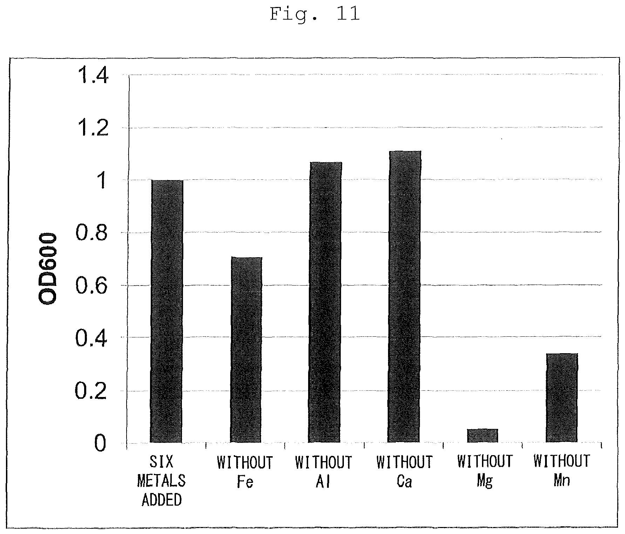

FIG. 11 illustrates the relative values of the turbidity (OD 600) of the culture broth 12 hours after the start of culturing of *Bacillus* A (the values relative to a system to which six metal components have been added).

As illustrated in FIG. 11, systems from which iron, magnesium, and manganese had been excluded had lower culture broth turbidity (OD 600) than in the system with six metal components added. It was therefore clear that these metal components are necessary to promote the proliferation of *Bacillus* A.

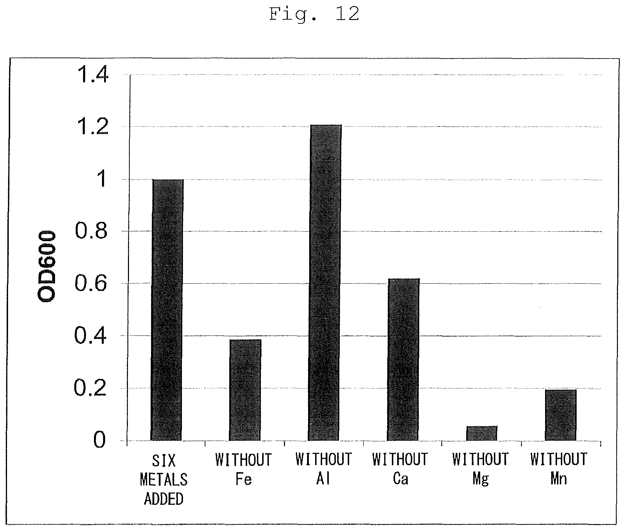

FIG. 12 illustrates the relative value of the turbidity (OD 600) of the culture broth 12 hours after the start of culture of *Bacillus* B (the value relative to a system with six metal components added).

As illustrated in FIG. 12, systems from which iron, calcium, magnesium, and manganese had been excluded had lower culture broth turbidity (OD 600) than the system with six metal components added. It was therefore clear that these metal components are necessary to promote the growth of *Bacillus* B.

Although not illustrated in the above results, manganese, when added in an excess amount, conversely had the operational effect of inhibiting the proliferation of *Bacillus.*

<Test Example 6> (Effect of mineral components on proteolytic enzyme production by *Bacillus* bacteria)

Iron, aluminum, calcium, and magnesium were selected as metal components other than silicon, which is commonly included in minerals, and the effect of each on the proteolytic enzyme production by *Bacillus* bacteria was studied. *Bacillus* A and *Bacillus* B were used as *Bacillus* bacteria in the same way as in Test Example 5.

Nutrient media were respectively inoculated with each *Bacillus,* and shake culturing was carried out at 30°C. Silicon (final concentration: 27.8 mg/L), iron (final concentration: 9 mg/L), aluminum (final concentration: 8.8 mg/L), calcium (final concentration: 17.2 mg/L), and magnesium (final concentration: 18 mg/L) were added to the media as metal components to yield the final concentration shown in parentheses. A system to which no specific metal components were added was also established.

Culturing was stopped 24 hours after the start of culture, and the proteolytic activity of the culture broth from which the *Bacillus* had been removed by centrifugation was measured in the same manner as in Test Example 3. It is noted that poliferation was found to reach saturation 24 hours after the start of culturing.

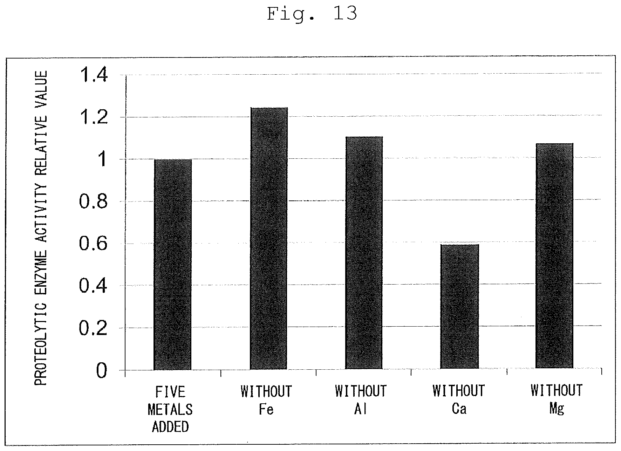

FIG. 13 illustrates the relative value of the proteolytic enzyme activity in the culture broth of *Bacillus* A per cell count (value per cell count relative to a system to which five metal components were added).

As illustrated in FIG. 13, the proteolytic enzyme activity decreased in a system from which calcium was excluded more than the system to which five metal components were added. It was therefore clear that calcium is necessary to promote the secretion of proteolytic enzymes from *Bacillus* A.

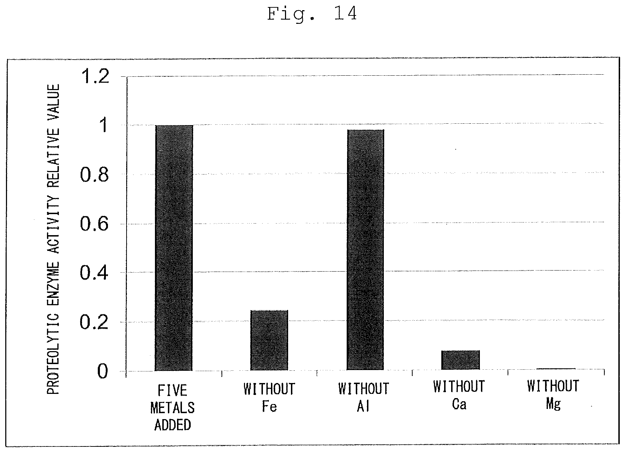

FIG. 14 illustrates the relative value of the proteolytic enzyme activity in the culture broth of *Bacillus* B per cell count (value per cell count relative to a system to which five metal components were added).

As illustrated in FIG. 14, the proteolytic enzyme activity decreased in systems from which iron, calcium, and magnesium were excluded more than the system to which five metal components were added. It was therefore clear that these metal components are necessary to promote the secretion of proteolytic enzymes from *Bacillus* B.

<Test Example 7> (Effect of mineral components on carbohydrate-degrading enzyme production by *Bacillus* bacteria)

The activity of carbohydrate-degrading enzymes secreted in the culture broth was studied in the same manner as in Test Example 4 using the respective culture broths of each *Bacillus,* which were cultured in the same manner as in Test Example 6.

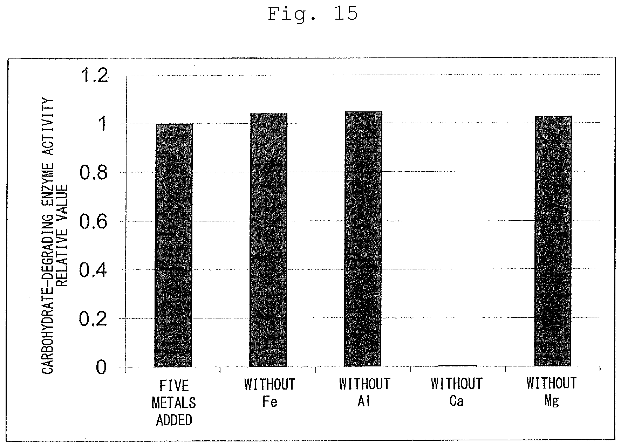

FIG. 15 illustrates the relative value of the carbohydrate-degrading enzyme activity in the culture broth of *Bacillus* A per cell count (value per cell count relative to the system to which five metal components were added).

As illustrated in FIG. 15, the carbohydrate-degrading enzyme was lower in the system from which calcium was excluded than in the system to which five metal components were added. It was therefore clear that calcium is necessary to promote the secretion of carbohydrate-degrading enzymes from *Bacillus* A.

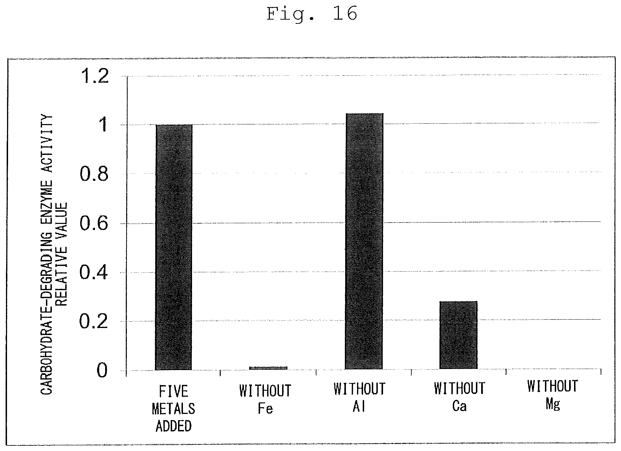

FIG. 16 illustrates the relative value of the carbohydrate-degrading enzyme activity in the culture broth of *Bacillus* B per cell count (value per cell count relative to system to which five metal components were added).

As illustrated in FIG. 16, the carbohydrate-degrading enzyme activity decreased in the systems from which iron, calcium, and magnesium were excluded more than the system to which five metal components were added. It was therefore clear that these metal components are necessary to promote the secretion of carbohydrate-degrading enzymes from *Bacillus* B.

As validated by the above Test Examples 5-7, it was clear that an activator to be used in the activated sludge treatment of wastewater in which *Bacillus* bacteria predominate preferably includes at least iron, magnesium, and calcium in addition to silicon known in the past. It was also clear that a combination of the above four metal components is preferably used, as appropriate, in accordance with the type of *Bacillus* bacteria.

Table 3 gives examples of compositions of various mineral-derived materials that have been widely used in the past for other applications.

[Table 3]

| Name of material | Mass% | | | | | |
|---|---|---|---|---|---|---|
| | Silicon | Iron | Calcium | Magnesium | Aluminum | Manganese |
| Blast furnace slag | 15.9 | 0.32 | 29.3 | 3.1 | 8.12 | 0.14 |
| Diatomaceous earth | 38.6 | 2.14 | 24.4 | 10.3 | 0.04 | 0.01 |
| Perlite | 19.3 | 3.62 | 3.68 | 4.21 | 3.44 | 0.08 |
| Cement | 22.6 | 3.3 | 64.7 | 2.7 | 5.7 | no data |

As illustrated in Table 3, in addition to the blast furnace slag validated in Test Examples 1-4, examples of mineral-derived materials that contain silicon, iron, magnesium, and calcium include diatomaceous earth, perlite, and cement.

Therefore, despite including components that activate the proliferation and enzyme activity of *Bacillus* bacteria, these mineral-derived materials are commonly sparingly-soluble materials. By pulverizing such materials to a predetermined particle size for use as activators in the same way as the blast furnace slag validated in Test Examples 1-4, it is thought that the activating effects involving *Bacillus* bacteria proliferation and enzyme activity will be demonstrated more prominently.

Moreover, by combining, as appropriate, the metal components validated in Test Examples 5-7, it is thought that the activating effects involving *Bacillus* bacteria proliferation and enzyme activity will be demonstrated more prominently.

[Explanation of Numerals and Characters]

| | |
|---|---|
| 1: | Primary settling basin |
| 2: | Treatment tank |
| 3: | Settling tank |
| 4: | Blower |
| 5: | Aeration adjustment valve |
| 6: | Diffuser plate |
| 7: | Sludge return pump |
| 8: | Sludge removal pump |
| 9: | Activator supply tank |
| 10: | Activator injection pump |
| 11: | Activator injection adjustment valve |
| 12: | Measuring instrument |
| 13: | Enzyme activity measuring instrument |
| 14: | Calculation unit |
| L1, L2, L3: | Pipe |

**Claims**

1. A method for treating wastewater in which wastewater including organic matter is introduced into a treatment tank and the wastewater is subjected to an activated sludge treatment by microorganisms in the treatment tank, wherein the method for treating wastewater is **characterized in that** an activator containing a component for activating the microorganisms is added to the wastewater to be subjected to the activated sludge treatment in the treatment tank, and at least 50% (by quantity) of the entirety of the activator has a particle size of less than 10 $\mu$m.

2. The method for treating wastewater according to claim 1, wherein the microorganisms include *Bacillus* bacteria.

3. The method for treating wastewater according to claim 1 or 2, wherein the activator includes a silicon compound.

4. The method for treating wastewater according to claim 3, wherein the activator also includes at least one selected from the group consisting of an iron compound, a magnesium compound, a calcium compound, and a manganese compound.

5. The method for treating wastewater according to any one of claims 1-4, wherein the activator is obtained by pulverizing at least one selected from the group consisting of blast furnace slag, diatomaceous earth, perlite, and cement.

6. The method for treating wastewater according to any one of claims 3-5, wherein the activator is used by having a solubilizer of a silicon compound additionally combined with the activator.

7. The method for treating wastewater according to claim 6, wherein the solubilizer includes a cationic polymer for suppressing polymerization of silicon and/or a silicon compound.

8. An activator to be used in a method for treating wastewater in which wastewater including organic matter is introduced into a treatment tank and the wastewater is subjected to an activated sludge treatment by microorganisms in the treatment tank, wherein the activator for treating wastewater is **characterized in** containing a component for activating the microorganisms, at least 50% (by quantity) of the entirety of the activator having a particle size of less than 10 μm.

9. The activator according to claim 8, wherein the microorganisms include *Bacillus* bacteria.

10. The activator according to claim 8 or 9, including a silicon compound as a component for activating the microorganisms.

11. The activator according to claim 10, further including at least one selected from the group consisting of an iron compound, a magnesium compound, a calcium compound, and a manganese compound as the component for activating the microorganisms.

12. The activator according to any one of claims 8-11, wherein the activator is obtained by pulverizing at least one selected from the group consisting of blast furnace slag, diatomaceous earth, perlite, and cement.

13. The activator according to any one of claims 10-12 used in combination with a solubilizer of a silicon compound.

14. The activator according to claim 13, wherein the solubilizer includes a cationic polymer for suppressing polymerization of silicon and/or a silicon compound.

Fig. 1

Fig. 2

```
                          ┌─────────────┐
                          │    START    │
                          └─────────────┘
                                 │
                                 ▼
                    ┌──────────────────────────┐
                    │       SAMPLING OF        │
                    │   REACTION TANK WATER    │
                    └──────────────────────────┘
                                 │
                                 ▼
                    ┌──────────────────────────┐
                    │  SOLID-LIQUID SEPARATION │
                    │          MEANS           │
                    └──────────────────────────┘
                                 │
                                 ▼
                    ┌──────────────────────────┐
                    │      COLLECTION OF       │
                    │      SAMPLE WATER        │
                    └──────────────────────────┘
                                 │
  PROTEASE DETECTION             │
      REAGENT ──────────┐        │
                        ▼        ▼
                    ┌──────────────────────────┐
                    │     PROTEASE REACTION     │
                    └──────────────────────────┘
                                 │
                                 ▼
                    ┌──────────────────────────┐
                    │      MEASUREMENT OF       │
                    │   FLUORESCENCE INTENSITY  │
                    └──────────────────────────┘
                                 │
                                 ▼
                    ┌──────────────────────────┐
                    │     PROTEASE ACTIVITY     │
                    │       COMPUTATION         │
                    └──────────────────────────┘
```

Fig. 3

```
                        ┌─────────────┐
                        │    START    │
                        └─────────────┘
                               │
                               ▼
                   ┌───────────────────────┐
                   │      SAMPLING OF       │
                   │  REACTION TANK WATER   │
                   └───────────────────────┘
                               │
                               ▼
                   ┌───────────────────────┐
                   │ SOLID-LIQUID SEPARATION│
                   │         MEANS          │
                   └───────────────────────┘
                               │
                               ▼
                   ┌───────────────────────┐
                   │COLLECTION OF SAMPLE WATER│
                   └───────────────────────┘
                               │
STARCH SOLUTION ────────┐      │
                        ▼      ▼
                   ┌───────────────────────┐
                   │   STARCH DEGRADATION   │
                   │       REACTION         │
                   └───────────────────────┘
                               │
IODINE SOLUTION ────────┐      │
                        ▼      ▼
                   ┌───────────────────────┐
                   │ IODINE-STARCH REACTION │
                   └───────────────────────┘
                               │
                               ▼
                   ┌───────────────────────┐
                   │MEASUREMENT OF ABSORBANCE│
                   └───────────────────────┘
                               │
                               ▼
                   ┌───────────────────────┐
                   │    AMYLASE ACTIVITY    │
                   │      COMPUTATION       │
                   └───────────────────────┘
```

Fig. 4

Fig. 5

Fig. 6

Fig. 7

Fig. 8

Fig. 9

Fig. 10

Fig. 11

Fig. 12

Fig. 13

Fig. 14

Fig. 15

Fig. 16

**INTERNATIONAL SEARCH REPORT**

International application No.

PCT/JP2016/056340

A. CLASSIFICATION OF SUBJECT MATTER

*C02F3/00*(2006.01)i, *C02F3/10*(2006.01)i, *C02F3/12*(2006.01)i, *C02F3/34*(2006.01)i

According to International Patent Classification (IPC) or to both national classification and IPC

B. FIELDS SEARCHED

Minimum documentation searched (classification system followed by classification symbols)
C02F3/00, C02F3/10, C02F3/12, C02F3/34

Documentation searched other than minimum documentation to the extent that such documents are included in the fields searched
Jitsuyo Shinan Koho                1922-1996   Jitsuyo Shinan Toroku Koho   1996-2016
Kokai Jitsuyo Shinan Koho   1971-2016   Toroku Jitsuyo Shinan Koho   1994-2016

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 09-187779 A  (Tsutomu NISHIMURA), 22 July 1997 (22.07.1997), claims 1, 5; paragraphs [0001], [0009], [0010] (Family: none) | 1-14 |
| Y | JP 07-136676 A  (Toyo Bio Reactor Yugen Kaisha), 30 May 1995 (30.05.1995), claims 1 to 4; paragraphs [0001], [0020] to [0023], [0035] (Family: none) | 1-14 |
| Y | JP 2012-005924 A  (Keiichiro ASAOKA), 12 January 2012 (12.01.2012), claims 1, 7 to 11; paragraph [0001] (Family: none) | 1-14 |

[X]  Further documents are listed in the continuation of Box C.          [ ]   See patent family annex.

| | | |
|---|---|---|
| * | Special categories of cited documents: | "T"   later document published after the international filing date or priority date and not in conflict with the application but cited to understand the principle or theory underlying the invention |
| "A" | document defining the general state of the art which is not considered    to be of particular relevance | |
| "E" | earlier application or patent but published on or after the international filing date | "X"   document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&"   document member of the same patent family |

| Date of the actual completion of the international search | Date of mailing of the international search report |
|---|---|
| 26 May 2016 (26.05.16) | 07 June 2016 (07.06.16) |

| Name and mailing address of the ISA/ | Authorized officer |
|---|---|
| Japan Patent Office 3-4-3,Kasumigaseki,Chiyoda-ku, Tokyo 100-8915,Japan | Telephone No. |

Form PCT/ISA/210 (second sheet) (January 2015)

**INTERNATIONAL SEARCH REPORT**

| International application No. |
|---|
| PCT/JP2016/056340 |

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2011-524248 A (Nalco Co.),<br>01 September 2011 (01.09.2011),<br>claims; paragraphs [0001], [0006]<br>& US 2009/0294374 A1<br>claims; paragraphs [0001], [0005]<br>& WO 2009/148978 A1    & EP 2303785 A1<br>& KR 10-2011-0018337 A   & CN 102046539 A<br>& TW 200951082 A      & RU 2010153577 A | 6-7,13-14 |
| Y | JP 58-074196 A (Calgon Corp.),<br>04 May 1983 (04.05.1983),<br>claims 1, 5<br>& EP 75514 A1<br>claims 1, 5<br>& AU 8838182 A | 6-7,13-14 |
| A | JP 2005-295887 A (Yugen Kaisha Chuo Bachiru World),<br>27 October 2005 (27.10.2005),<br>claims; paragraph [0001]<br>(Family: none) | 1-14 |
| A | JP 2004-344886 A (Koa Corp.),<br>09 December 2004 (09.12.2004),<br>claims; paragraphs [0001], [0016]<br>(Family: none) | 1-14 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002113486 A
- JP 2005329301 A
- JP 2012005924 A

**Non-patent literature cited in the description**

- *Journal of Environmental Biotechnology,* 2011, vol. 11 (1-2), 47-53
- *Abstracts of the 65th Meeting of the Society for Biotechnology,* 221